# EUROPEAN PATENT APPLICATION

(11) **EP 4 721 613 A1**
(43) Date of publication of application: **08.04.2026**
(21) Application number: 24825717.2
(22) Date of filing: 05.06.2024
(51) Int. Cl.: A41B 9/12, A61F 13/49, A61F 13/535, A61F 13/539

(54) **WATER-ABSORBING SANITARY UNDERWEAR**

(30) Priority: 20.06.2023 JP 2023101024
(71) Applicant: Unicharm Corporation, Shikokuchuo-shi, Ehime 799-0111 (JP)
(72) Inventor: MATSUI, Saeko, Kanonji-shi, Kagawa 769-1602 (JP); TAMURA, Tatsuya, Kanonji-shi, Kagawa 769-1602 (JP); MARUYAMA, Takashi, Kanonji-shi, Kagawa 769-1602 (JP); KOKUDO, Yumiko, Kanonji-shi, Kagawa 769-1602 (JP)
(74) Representative: Dolleymores
(86) International application number: PCT/JP2024/020462
(87) International publication number: WO 2024/262310

(57) **Abstract**

[SUMMARY] To provide absorbent sanitary shorts capable of suppressing leakage of body fluid while enhancing fit to a body in a crotch region. The absorbent sanitary shorts (1) are reusable shorts comprising a surface layer (10), an absorbent layer (20) arranged on a non-skin side (T2) of the surface layer, and a waterproof layer (30) arranged on the non-skin side of the absorbent layer. The shorts comprise a crotch region (CR) arranged between a front end edge of the absorbent layer and a rear end edge of the absorbent layer, a crotch center (CRC) located at a center in a width direction and a center in a front-rear direction of the crotch region, and a pressing portion (60) arranged on the non-skin side relative to a non-skin surface of the absorbent layer. The crotch region is provided with crotch joining portions (40) where only the surface layer and the absorbent layer are joined among the surface layer, the absorbent layer, and the waterproof layer, and a crotch non-joining portion (45) where the surface layer, the absorbent layer, and the waterproof layer are not joined to each other. The crotch joining portions are arranged on both sides of a first direction, which is one of the front-rear direction and the width direction, across the crotch center. The crotch non-joining portion is arranged overlapping with the pressing portion between the crotch joining portions in the first direction.

## Description

### [TECHNICAL FIELD]

The present invention relates to reusable absorbent sanitary shorts.

### [BACKGROUND ART]

Patent Literature 1 discloses reusable absorbent sanitary shorts. The absorbent sanitary shorts in Patent Literature 1 include a surface layer, an absorbent layer, and a waterproof layer, and are configured such that body fluid can be absorbed by the absorbent layer. The surface layer, the absorbent layer, and the waterproof layer are joined at joining portions. The joining portions are formed at an interval in a width direction in a crotch region arranged to face a vaginal opening, and to extend in a front-rear direction.

### [CITATION LIST]

### [PATENT LITERATURE]

[PATENT LITERATURE 1] Japanese Unexamined Patent Application Publication No. 2023-037804

### [SUMMARY OF INVENTION]

In the absorbent sanitary shorts in Patent Literature 1, the surface layer, the absorbent layer, and the waterproof layer are sewn together at the joining portions. In the sewn portions, body fluid absorbed by the surface layer and the absorbent layer is likely to reach the waterproof layer. Furthermore, in the waterproof layer, holes through which threads pass are formed by sewing. Thus, the body fluid may reach a non-skin side beyond the waterproof layer through the holes, and leakage may be caused.

The present invention has been made in view of such problems, and an object thereof is to provide absorbent sanitary shorts capable of suppressing leakage of body fluid while enhancing fitness to a body in a crotch region.

Absorbent sanitary shorts according to one aspect are reusable shorts comprising: a surface layer; an absorbent layer arranged on a non-skin side relative to the surface layer; and a waterproof layer arranged on the non-skin side relative to the absorbent layer. The shorts comprise: a crotch region arranged between a front end edge of the absorbent layer and a rear end edge of the absorbent layer; a crotch center located at a center in the width direction and a center in the front-rear direction of the crotch region; and a pressing portion arranged on the non-skin side relative to a non-skin surface of the absorbent layer. The crotch region is provided with: crotch joining portions where only the surface layer and the absorbent layer are joined among the surface layer, the absorbent layer, and the waterproof layer; and a crotch non-joining portion where the surface layer, the absorbent layer, and the waterproof layer are not joined to each other. The crotch joining portions are arranged on both sides of a first direction, which is one of the front-rear direction and the width direction, across the crotch center. The crotch non-joining portion is arranged overlapping with the pressing portion between the crotch joining portions in the first direction. According to this aspect, the provision of the crotch joining portions can suppress displacement of the surface layer that contacts the body in the crotch region, thereby improving the fit of the surface layer. Since the crotch joining portions are arranged on both sides of the crotch center in the first direction, the surface layer is prevented from shifting relative to the absorbent layer on both sides of the crotch center, which can suppress displacement of the surface layer at the crotch center. Furthermore, the crotch joining portions have higher rigidity compared to the crotch non-joining portion. Therefore, providing the crotch joining portions can suppress twisting of the crotch region, keep the crotch region against the body, and suppress leakage of body fluid. Moreover, the crotch non-joining portion arranged between the crotch joining portions tends to rise toward the wearer's side with the crotch joining portions as a base, and further, the pressing portion pushes the absorbent layer and the surface layer up toward the body, enhancing the fit to the body. The crotch joining portion joins only the surface layer and the absorbent layer among the surface layer, the absorbent layer, and the waterproof layer. That is, the crotch joining portion does not join the waterproof layer. Therefore, it is possible to suppress body fluid absorbed by the surface layer and the absorbent layer from reaching the waterproof layer via the crotch joining portion, thereby suppressing leakage of body fluid.

### [BRIEF DESCRIPTION OF DRAWINGS]

[FIG. 1] FIG. 1 is a plan view of absorbent sanitary shorts according to an embodiment as seen from a front side.
[FIG. 2] FIG. 2 is a plan view of the absorbent sanitary shorts according to the embodiment as seen from a rear side.
[FIG. 3] FIG. 3 is a plan view of a crotch region of the absorbent sanitary shorts according to the embodiment as seen from a skin side.
[FIG. 4] FIG. 4 is a schematic cross-sectional view showing the crotch region at a cross section along line A-A shown in FIG. 3.
[FIG. 5] FIG. 5 is a schematic cross-sectional view schematically showing a cross section along line D-D shown in FIG. 3.
[FIG. 6] FIG. 6 is a schematic enlarged view in which part of the absorbent sanitary shorts is enlarged.
[FIG. 7] FIG. 7 is a diagram schematically showing a deformed state during wearing based on the cross section shown in FIG. 4.

### [DESCRIPTION OF EMBODIMENTS]

### (1) Outline of embodiment

According to description in the present specification and the attached drawings, at least the following matters become apparent.

An invention according to Aspect 1 is reusable absorbent sanitary shorts. The absorbent sanitary shorts comprise a surface layer, an absorbent layer arranged on a non-skin side relative to the surface layer, and a waterproof layer arranged on the non-skin side relative to the absorbent layer. The shorts comprise a crotch region arranged between a front end edge of the absorbent layer and a rear end edge of the absorbent layer, a crotch center located at a center in the width direction and a center in the front-rear direction of the crotch region, and a pressing portion arranged on the non-skin side relative to a non-skin surface of the absorbent layer to press the absorbent layer toward a skin side. The crotch region is provided with crotch joining portions where only the surface layer and the absorbent layer are joined among the surface layer, the absorbent layer, and the waterproof layer, and a crotch non-joining portion where the surface layer, the absorbent layer, and the waterproof layer are not joined to each other. The crotch joining portions are arranged on both sides of a first direction, which is one of the front-rear direction and the width direction, across the crotch center. The crotch non-joining portion is arranged overlapping with the pressing portion between the crotch joining portions in the first direction. According to this aspect, the provision of the crotch joining portions can suppress displacement of the surface layer that contacts the body in the crotch region, thereby improving the fit of the surface layer. Since the crotch joining portions are arranged on both sides of the crotch center in the first direction, the surface layer is prevented from shifting relative to the absorbent layer on both sides of the crotch center, which can suppress displacement of the surface layer at the crotch center. Furthermore, the crotch joining portions have higher rigidity compared to the crotch non-joining portion. Therefore, providing the crotch joining portions can suppress twisting of the crotch region, keep the crotch region against the body, and suppress leakage of body fluid. Moreover, the crotch non-joining portion arranged between the crotch joining portions tends to rise toward the wearer's side with the crotch joining portions as a base, and further, the pressing portion pushes the absorbent layer and the surface layer up toward the body, enhancing the fit to the body. The crotch joining portion joins only the surface layer and the absorbent layer among the surface layer, the absorbent layer, and the waterproof layer. That is, the crotch joining portion does not join the waterproof layer. Therefore, it is possible to suppress body fluid absorbed by the surface layer and the absorbent layer from reaching the waterproof layer via the crotch joining portion, thereby suppressing leakage of body fluid.

According to a preferred aspect, an invention according to Aspect 2 may have the following feature in the invention according to Aspect 1. The first direction is the width direction. According to this aspect, a convex shape that protrudes toward the skin side can be formed in the center of the crotch region in the width direction, with the crotch joining portions located on both sides of the crotch center in the width direction as a base. This convex shape can improve the fit to the vaginal opening, enhance the wicking of body fluid, and suppress side leakage.

According to a preferred aspect, an invention according to Aspect 3 may have the following feature in the invention according to Aspect 2. The crotch joining portion and the crotch non-joining portion extend in the front-rear direction. According to this aspect, since the crotch joining portion and the crotch non-joining portion extend in the front-rear direction, the fit to the body can be improved over a continuous range in the front-rear direction. Furthermore, the crotch joining portions extending in the front-rear direction serve as a continuous base, making it easy to raise the region sandwiched by the crotch joining portions in the width direction toward the skin side and to form a continuous convex shape in the front-rear direction. Moreover, as this convex shape propagates rearward, it improves the fit to the gluteal cleft and can also suppress rear leakage.

According to a preferred aspect, an invention according to Aspect 4 may have the following feature in the invention according to Aspect 3. The crotch joining portion is continuous from the front end edge of the crotch region to the rear end edge of the crotch region. According to this aspect, the surface layer and the absorbent layer are joined by the crotch joining portion over the entire area of the crotch region in the front-rear direction, thereby suppressing twisting of the surface layer and the absorbent layer. By suppressing twisting of the surface layer, the surface layer can be kept in contact with the wearer's crotch to ensure the wicking of body fluid. Additionally, by suppressing twisting of the absorbent layer, the absorption area for body fluid can be secured.

According to a preferred aspect, an invention according to Aspect 5 may have the following feature in the invention according to Aspect 3 or Aspect 4. The pressing portion extends in the front-rear direction. According to this aspect, since the pressing portion, the crotch joining portion, and the crotch non-joining portion extend in the same front-rear direction, a convex shape can be formed over a continuous range in the front-rear direction, thereby further improving the fit to the body.

According to a preferred aspect, an invention according to Aspect 6 may have the following feature in the invention according to any one of Aspects 1 to 5. The pressing portion is arranged at the center of the crotch region in the width direction. According to this aspect, the center of the crotch region in the width direction is pushed up toward the skin side by the pressing portion. Therefore, the fit to the vaginal opening can be further improved, and the fit to the gluteal cleft can also be improved.

According to a preferred aspect, an invention according to Aspect 7 may have the following feature in the invention according to Aspect 5 or Aspect 6. A front end edge of the pressing portion is located on a rear side relative to a front end edge of the crotch region. According to this aspect, the front end edge of the pressing portion is located on the rear side relative to the front end edge of the crotch region, and a region that does not overlap with the pressing portion is provided in a front part of the absorbent layer. The front part of the absorbent layer is less likely to be positioned opposite the excretion opening and has less need to be pressed against the body. By making the front part of the absorbent layer relatively flat, the body can be covered by the absorbent layer, and the body can be covered over a surface by the absorbent layer. In the region where the absorbent layer and the pressing portion overlap, it is pressed against the body side by the expansion and contraction of the pressing portion, which can reduce the gap between the body and the shorts. Furthermore, since wrinkles due to the expansion and contraction of the pressing portion are less likely to form in the front part of the crotch region, the aesthetic appearance of the shorts can also be improved.

According to a preferred aspect, an invention according to Aspect 8 may have the following feature in the invention according to any one of Aspects 5 to 7. A rear end edge of the pressing portion is located on a rear side relative to a rear end edge of the crotch region. According to this aspect, since the pressing portion extends to the rear side of the rear end edge of the absorbent layer, the rear end edge of the absorbent layer can be fitted to the body, thereby suppressing rear leakage.

According to a preferred aspect, an invention according to Aspect 9 may have the following feature in the invention according to any one of Aspects 1 to 8. The 50% tensile strength of the waterproof layer in the crotch region is lower than the 50% tensile strength of the surface layer in the crotch region, and is lower than the 50% tensile strength of the absorbent layer in the crotch region. According to this aspect, since the waterproof layer is easily stretchable, even when the shorts are significantly stretched, such as during exercise, the waterproof layer can follow the body's movements and remain along the body, thereby suppressing leakage of body fluid. On the other hand, since the surface layer and the absorbent layer are less stretchable, they can cover the body while maintaining the fitted shape formed in a relatively less stretched state (a state stretched from 1.0 to 1.1 times) when significantly stretched, thereby maintaining absorption capacity and suppressing leakage.

According to a preferred aspect, an invention according to Aspect 10 may have the following feature in the invention according to any one of Aspects 1 to 9. The crotch region includes a crotch center region located at the center among three equally divided regions obtained by dividing the crotch region in the front-rear direction. The 10% tensile strength of the surface layer in the crotch center region is lower than the 10% tensile strength of the absorbent layer in the crotch center region, and is lower than the 10% tensile strength of the waterproof layer in the crotch center region. The crotch center region particularly requires a good fit to the excretion opening, and it is important to fit it into the gaps of the body. The surface layer is easily stretchable in the crotch center region and readily follows the body's movements. The surface layer is easily deformed into a convex shape by the expansion and contraction of the pressing portion and tends to maintain a continuous fit against the vaginal opening. The waterproof layer and the absorbent layer are less stretchable in the crotch center region and can support the surface layer from the non-skin side relative to the surface layer, maintaining the shape of the surface layer and suppressing leakage.

According to a preferred aspect, an invention according to Aspect 11 may have the following feature in the invention according to any one of Aspects 1 to 10. The crotch region includes a crotch rear region located on a rear side among three equally divided regions obtained by dividing the crotch region in the front-rear direction. The 50% tensile strength of the waterproof layer in the crotch rear region is lower than the 50% tensile strength of the surface layer in the crotch rear region. The 50% tensile strength of the surface layer in the crotch rear region is lower than the 50% tensile strength of the absorbent layer in the crotch rear region. The crotch rear region is an area that is easily deformed by the movement of the buttocks. Since the waterproof layer is easily stretchable, it can follow the body's movements, maintain a good fit to the buttocks, and suppress rear leakage. Furthermore, the surface layer is more stretchable than the absorbent layer and readily follows the body's movements. The absorbent layer can maintain its shape, continue to cover the body, and suppress leakage.

According to a preferred aspect, an invention according to Aspect 12 may have the following feature in the invention according to any one of Aspects 1 to 11. The crotch joining portion is a sewn portion in which the surface layer and the absorbent layer are sewn together. According to this aspect, the surface layer and the absorbent layer are brought into close contact by threads or the like at the sewn portion, and the surrounding area rises in the thickness direction relative to the sewn portion. Therefore, the crotch joining portion, being a sewn portion, is more likely to serve as a base, facilitating the formation of a shape where the crotch non-joining portion between the crotch joining portions rises further toward the wearer's side. Furthermore, since holes for threads to pass through are formed in the sewn portion, the wicking of body fluid from the surface layer to the absorbent layer can be enhanced, improving the wearing sensation.

According to a preferred aspect, an invention according to Aspect 13 may have the following feature in the invention according to any one of Aspects 1 to 11. The crotch joining portion is a bonded portion where a non-skin surface of the surface layer and a skin surface of the absorbent layer are bonded. Since the bonded portion adheres the non-skin surface of the surface layer to the skin surface of the absorbent layer and is not constrained from the skin surface of the surface layer to the non-skin surface of the absorbent layer, the degrees of freedom for the skin surface of the surface layer and the non-skin surface of the absorbent layer are increased. Therefore, it can easily follow the body's movements and readily achieve a good fit to the body and the effect of continuously covering the body. Furthermore, a bonded portion made of adhesive is likely to have a larger area compared to a sewn portion. Accordingly, the area of the joining portion can be secured, the surface layer and the absorbent layer can be joined over a wide range, and twisting and displacement can be suppressed.

According to a preferred aspect, an invention according to Aspect 14 may have the following feature in the invention according to any one of Aspects 1 to 13. A surface waterproof layer that covers an outer edge part of the surface layer is disposed in the crotch region. According to this aspect, the skin side of the surface layer can be covered by the surface waterproof layer, and the non-skin side of the surface layer can be covered by the waterproof layer, thereby suppressing leakage of body fluid from the surface layer to the skin side and from the surface layer to the non-skin side.

According to a preferred aspect, an invention according to Aspect 15 may have the following feature in the invention according to Aspect 14. The surface waterproof layer includes a first waterproof layer constituting a skin surface of the shorts, and a second waterproof layer that is continuous with the first waterproof layer via a fold line and is located on a non-skin side relative to the first waterproof layer. An outer edge joining portion, where the surface layer, the absorbent layer, and the waterproof layer are joined, is disposed at an outer edge part of the crotch region. The outer edge joining portion includes a first outer edge joining portion where both the first waterproof layer and the second waterproof layer are joined, and a second outer edge joining portion where only the second waterproof layer of the first waterproof layer and the second waterproof layer is joined. The first outer edge joining portion and the second outer edge joining portion are sewn portions where the surface layer, the absorbent layer, and the waterproof layer are sewn together. According to this aspect, by providing the second outer edge joining portion, holes for threads or the like are not formed, making it difficult for body fluid once wicked into the absorbent layer to return to the surface waterproof layer side, thereby suppressing liquid returning. On the other hand, the first outer edge joining portion has holes formed therein, which can improve wicking from the surface waterproof layer side to the absorbent layer side and enhance breathability. By providing both the first outer edge joining portion and the second outer edge joining portion, it is possible to improve the wicking of body fluid, the effect of suppressing liquid returning, and breathability.

According to a preferred aspect, an invention according to Aspect 16 may have the following feature in the invention according to Aspect 15. The first outer edge joining portion is arranged at a front end part of the crotch region so as to straddle the center of the crotch region in the width direction. The first outer edge joining portion sews the first waterproof layer and is visually recognizable from the skin side of the shorts. The front end part of the crotch region is easy for the wearer to see when aligning during wearing, and the first outer edge joining portion can be used as a marker for alignment. In particular, when using a pad such as a napkin in combination with the absorbent sanitary shorts, the pad can also be aligned.

According to a preferred aspect, an invention according to Aspect 17 may have the following feature in the invention according to any one of Aspects 1 to 16. The absorbent sanitary shorts comprise an interior material including the surface layer, the absorbent layer, and the waterproof layer, and an exterior material arranged on a non-skin side relative to the interior material. The pressing portion is provided on the exterior material. The 10% tensile strength of the exterior material in the crotch region is lower than the 10% tensile strength of the interior material in the crotch region. According to this aspect, the exterior material is easily stretchable, and in a low-stretch state at the initial stage of wearing, the pressing portion provided on the exterior material presses the absorbent layer, allowing the convex portion to fit against recessed parts of the body such as the vaginal opening and buttocks, thereby maintaining a structure that is less prone to leakage.

According to a preferred aspect, an invention according to Aspect 18 may have the following feature in the invention according to Aspect 17. The pressing portion is constituted of a non-elastic member arranged on a non-skin side relative to the absorbent layer. Since the pressing portion is constituted of a non-elastic member and the exterior material provided with the pressing portion is easily stretchable, the region where the non-elastic member is present can press the absorbent layer toward the skin side due to the expansion and contraction of the exterior material, allowing for a fit with a convex shape.

According to a preferred aspect, an invention according to Aspect 19 may have the following feature in the invention according to any one of Aspects 1 to 17. The pressing portion is constituted of an elastic member arranged on a non-skin side relative to the absorbent layer. The expansion and contraction of the elastic member can press the absorbent layer toward the skin side, allowing the convex portion to fit to the body.

### (2) Absorbent sanitary shorts according to embodiment

The following describes absorbent sanitary shorts according to the embodiment (hereinafter, referred to as shorts 1) with reference to the drawings. In the description of the drawings below, the same or similar parts are indicated with the same or similar reference numerals. It should be noted that the drawings are schematic and that proportions of the dimensions and the like may be different from those in reality. Thus, specific dimensions and the like should be determined by taking the following explanations into consideration. Further, there may be some parts where relationships and proportions of dimensions vary among the drawings. The shorts 1 are shorts to be worn during menstruation or when caring for urinary incontinence or the like, and are configured to be repeatedly usable by washing. That is, the shorts are not disposable shorts but are reusable shorts. The shorts are configured to absorb body fluid excreted from a wearer. Examples of the body fluid to be absorbed include menstrual blood, urine, and vaginal discharge. The shorts do not necessarily include pulp and SAP as in disposable absorbent articles.

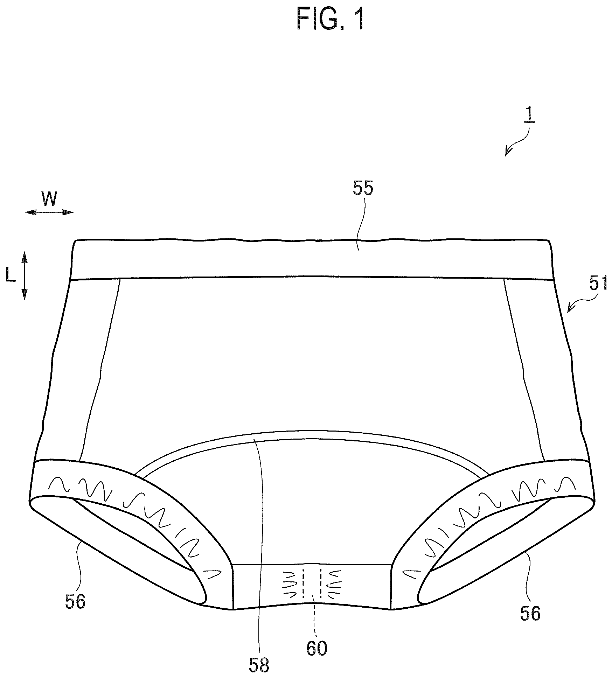

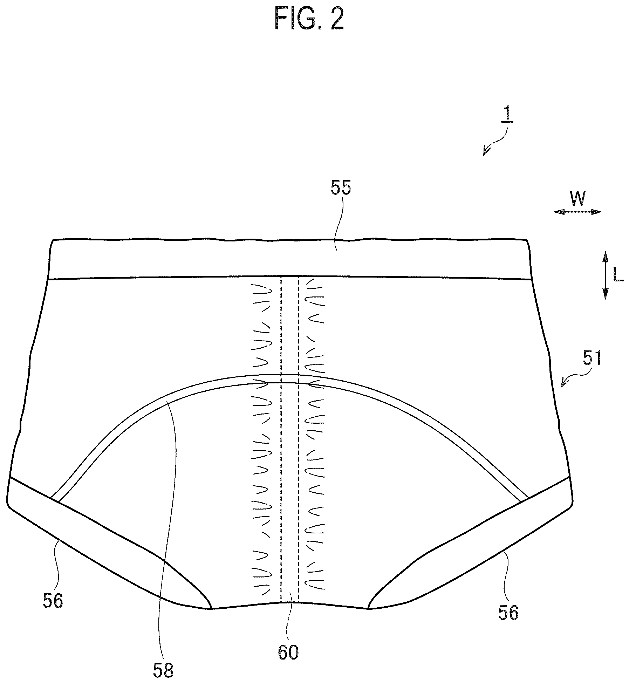

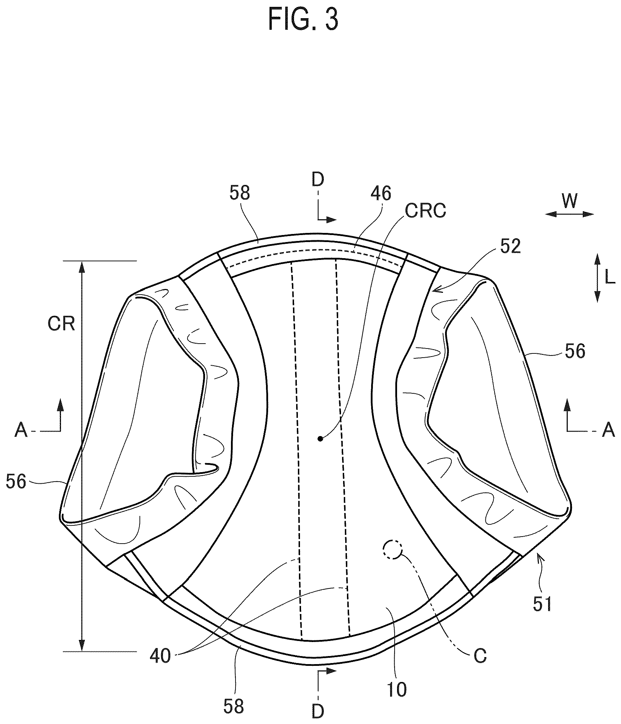

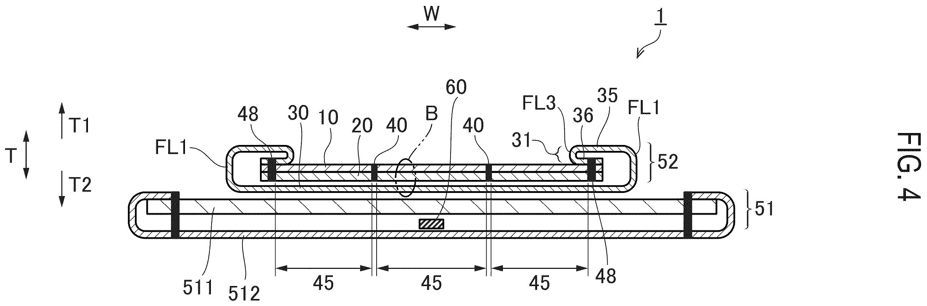

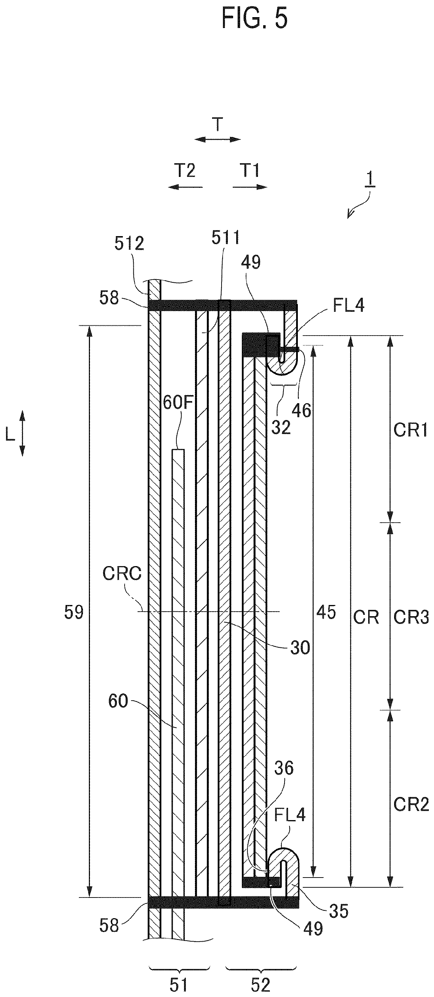

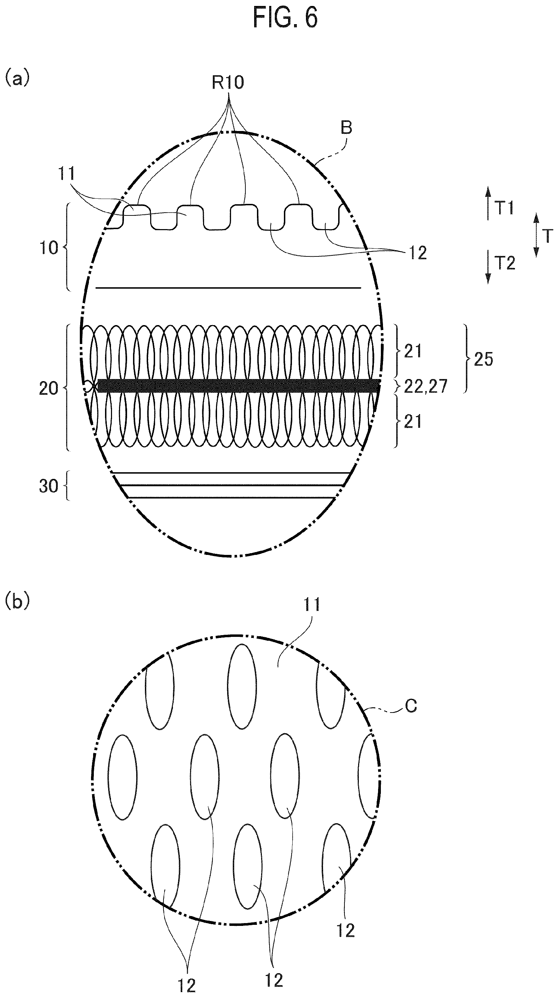

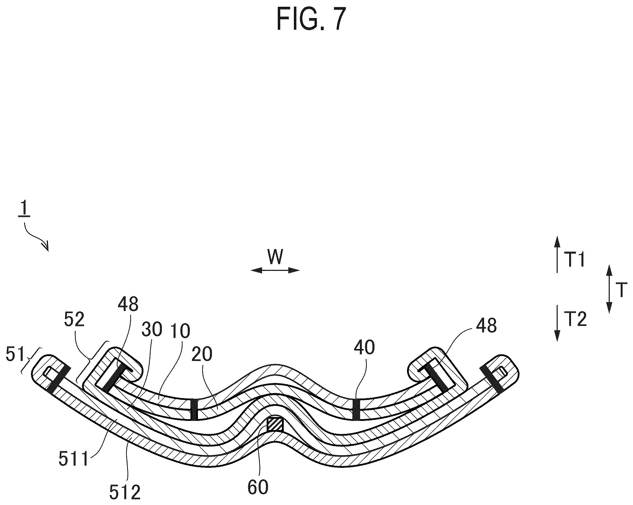

FIG. 1 and FIG. 2 are plan views of the shorts 1. FIG. 1 is a plan view as seen from a front side, and FIG. 2 is a plan view as seen from a rear side. FIG. 3 is a plan view of a crotch region CR of the shorts 1 as seen from a skin side, which shows the crotch region in a state in which an inner surface side of the shorts is rolled back toward an outer surface side. FIG. 4 is a cross-sectional view of the crotch region along line A-A shown in FIG. 3. FIG. 5 is a cross-sectional view of the crotch region along line D-D shown in FIG. 3. FIG. 6 is a schematic enlarged view in which part of the shorts is enlarged, and FIG. 6(a) is a schematic enlarged view schematically showing a part B in FIG. 4. FIG. 6(b) is a schematic enlarged view schematically showing a part C in FIG. 3, which is a schematic plan view showing a structure of a surface layer. FIG. 7 is a diagram schematically showing a deformed state during wearing based on the cross section shown in FIG. 4. It is to be noted that, for convenience of illustration, the drawings include portions in which respective constituent elements are illustrated as being separated from each other even at locations where the constituent elements are in contact with each other.

An outer side portion in the present invention refers to a part that extends over a certain range in a width direction W, including an outside edge in the width direction W. The outside edge refers to an outside edge in the width direction W. Further, the outside edge of any constituent element extending over a certain range in the front-rear direction refers to an edge obtained by connecting points positioned on outer sides, in the width direction, of the constituent element, throughout the entire constituent element. An inner side portion in the present invention refers to a part that extends over a certain range in the width direction W, including an interior edge in the width direction W. An inside edge refers to an interior edge in the width direction W. Further, the inside edge of any constituent element extending over a certain range in the front-rear direction refers to an edge obtained by connecting points positioned on inner sides, in the width direction, of the constituent element, throughout the entire constituent element. The front end and the rear end in the present invention each refer to a part that extends over a certain range in a front-rear direction L, including an edge in the front-rear direction L. The front end edge and the rear end edge each refer to an edge in the front-rear direction L. An outer end includes the front end and the rear end. An outer end edge includes the front end edge and the rear end edge. The outer edge is a concept including the outer end edge and the outside edge, and an outer edge part is a concept including the outer end and the outer side portion. Further, an inner lateral side is a side that extends along the front-rear direction L, while including the inside edge. An outer lateral side is a side that extends along the front-rear direction L, while including the outside edge. In the present specification, the expression "along the front-rear direction L" denotes a direction oriented at an angle smaller than 45° with respect to the front-rear direction L. The expression "along the width direction W" denotes a direction oriented at an angle smaller than 45° with respect to the width direction W.

The shorts 1 may have the front-rear direction L along a front-rear direction of a body, the width direction W orthogonal to the front-rear direction L, and a thickness direction T. The thickness direction T is a direction extending to a skin side (inner side) T1 toward the wearer side, and a non-skin side (outer side) T2 toward an opposite side of the wearer. A plane direction of the shorts 1 is a direction extending in the front-rear direction L and the width direction W. The shorts 1 may include an exterior material 51 that covers a waist of the wearer, and an interior material 52 that is placed against an excretion opening to absorb body fluid. The exterior material 51 is arranged on the non-skin side T2 relative to the interior material 52, covers the body of the wearer, and has a function of bringing the interior material 52 into contact with the body. The exterior material 51 includes a waist portion 55 that covers the waist of the wearer, and leg portions 56 that cover legs of the wearer. The exterior material 51 may be constituted of fabric, for example, but a material thereof is not particularly limited. For example, fabric containing nylon and polyurethane can be exemplified. The exterior material 51 in the present embodiment includes a first exterior material 511, a second exterior material 512 located on the non-skin side T2 relative to the first exterior material 511, and a pressing portion 60 located between the first exterior material 511 and the second exterior material 512.

The interior material 52 is arranged on the skin side T1 relative to the exterior material 51, and has a function of absorbing body fluid. The interior material 52 at least includes a surface layer 10, and an absorbent layer 20 arranged on the non-skin side T2 relative to the surface layer 10. The interior material 52 may include a waterproof layer 30 arranged on the non-skin side T2 relative to the absorbent layer 20. That is, the interior material 52 may be constituted of the surface layer 10, the absorbent layer 20, and the waterproof layer 30. The interior material 52 may be integrated by a side part joining portion 48 described later. In another form, the waterproof layer 30 may be disposed in the exterior material 51. The surface layer 10 is located to be closest to the skin side T1 in the interior material 52, and comes into contact with the wearer. The absorbent layer 20 absorbs (retains) body fluid guided from the surface layer 10. The absorbent layer 20 contains fibers, and is configured to temporarily absorb body fluid by retaining the body fluid in gaps among the fibers. The body fluid among the fibers is removed by washing the shorts 1, and moisture among the fibers is evaporated by drying the shorts, so that the absorbent layer 20 is configured to return to a state before absorbing the body fluid. The waterproof layer 30 may be a liquid-impermeable sheet, and may be mainly constituted of non-absorbent fibers.

The non-absorbent fibers will be described in more detail later. The waterproof layer 30 is arranged to cover the entire non-skin surface of the absorbent layer 20. The waterproof layer 30 in the present embodiment is constituted of a polyester sheet and a polyurethane sheet laminated on the polyester sheet. The waterproof layer 30 may be arranged to directly cover the non-skin surface of the absorbent layer 20, or may indirectly cover the absorbent layer 20 via another member.

The shorts 1 may include the crotch region CR arranged between a front end edge of the absorbent layer 20 and a rear end edge of the absorbent layer 20, a front-side region located on a front side relative to the crotch region CR, and a rear-side region located on a rear side relative to the crotch region. The shorts 1 may include a crotch center CRC. The crotch region CR is a region that comes into contact with an excretion opening of urine or menstrual blood of the wearer, and is a region to be placed against a crotch of the wearer. A range in the front-rear direction L of the crotch region CR may be a range in which the absorbent layer 20 is arranged. That is, a front end edge of the crotch region CR matches the front end edge of the absorbent layer 20, and a rear end edge of the crotch region CR matches the rear end edge of the absorbent layer 20. The crotch region CR may include a crotch center region CR3 located at a center among three equally divided regions obtained by dividing the crotch region CR in the front-rear direction L, a crotch front region CR1 located on a front side among the three equally divided regions, and a crotch rear region CR2 located on a rear side among the three equally divided regions. The three equally divided regions may be three equally divided regions obtained by dividing a length of the crotch region CR at the center in the width direction W. FIG. 5 shows ranges in the front-rear direction L of the crotch region CR, the crotch front region CR1, the crotch rear region CR2, and the crotch center region CR3. The crotch center CRC is located at the center in the width direction W and the center in the front-rear direction L of the crotch region CR, and is located within the crotch center region CR3. The interior material 52 may be arranged in the crotch region CR of the shorts 1. The interior material 52 and the exterior material 51 are arranged in the crotch region CR, and on the front side and the rear side relative to the crotch region CR, the interior material 52 is not arranged and only the exterior material 51 may be arranged.

The entire surface of the interior material 52 is not necessarily joined to the exterior material 51. As shown in FIG. 5, there may be disposed exterior joining portions 58 at which the interior material 52 is joined to the exterior material 51, and an exterior non-joining portion 59 at which the interior material 52 and the exterior material 51 are not joined but overlapped with each other. The exterior joining portions 58 in the present embodiment are disposed at a crotch front edge portion including the front end edge of the crotch region CR and extending in the width direction, and a crotch rear edge portion including the rear end edge of the crotch region CR and extending in the width direction W. The exterior non-joining portion 59 is present between the exterior joining portion 58 at the crotch front end and the exterior joining portion 58 at the crotch rear end.

The shorts 1 include the pressing portion 60 that is arranged on the non-skin side T2 relative to the non-skin surface of the absorbent layer 20, and presses the absorbent layer 20 toward the skin side T1. The pressing portion 60 may be arranged along the front-rear direction L or the width direction W. The pressing portion 60 is a portion that is arranged on the non-skin side T2 relative to the absorbent layer 20, and presses the absorbent layer 20 from the non-skin side T2 toward the skin side T1. The pressing portion 60 may be constituted of a member having elasticity such as a rubber thread, flat rubber having a predetermined width, or an elastic sheet, may be constituted of a combination of a non-elastic member having no elasticity (for example, a non-elastic string) and an elastic sheet arranged on the non-skin side relative to the non-elastic member, or may be constituted of a portion obtained by applying a material having elasticity (for example, urethane) to the exterior material 51. The pressing portion 60 may be attached to the interior material 52 (for example, the waterproof layer 30), or may be attached to the exterior material 51. The pressing portion 60 may be rubber containing nylon and polyurethane.

The pressing portion 60 may be constituted of an elastic member arranged on the non-skin side T2 relative to the absorbent layer 20. By expansion and contraction of the elastic member, the absorbent layer can be pressed toward the skin side to fit a protruding portion to the body. In another form, the pressing portion may be constituted of a non-elastic member arranged on the non-skin side relative to the absorbent layer. When the pressing portion is constituted of a non-elastic member, the pressing portion is disposed in the exterior material, and 10% tensile strength of the exterior material in the crotch region may be lower than 10% tensile strength of the interior material in the crotch region. With this configuration, the pressing portion is constituted of the non-elastic member, and the exterior material provided with the pressing portion is likely to stretch, so that a region where the non-elastic member is present can press the absorbent layer toward the skin side to be fitted in a protruding shape by expansion and contraction of the exterior material. In a form in which the pressing portion is constituted of the non-elastic member, preferably, a region having elasticity of the sheet may be arranged to cover the non-skin surface of the non-elastic member, and the non-elastic member may be pressed by the region having elasticity. As another form, the non-skin surface of the non-elastic member may be covered by the region having non-elasticity of the sheet, and the non-elastic member may be pressed by the region having elasticity continuous to a region having non-elasticity.

Subsequently, the following describes a configuration for improving diffusibility of body fluid in the shorts 1 configured as described above and for suppressing liquid returning in detail. The surface layer 10 and the absorbent layer 20 mainly contain non-absorbent fibers. Herein, the non-absorbent fibers are fibers that hardly absorb moisture, and include fibers that absorb a small amount of moisture. Specifically, the non-absorbent fibers are fibers having an official moisture regain of 5% or less. The official moisture regain is a value indicating a moisture regain in fibers in an environment at a temperature of 20°C and humidity of 65%. Examples of fibers having the official moisture regain of 5% or less may include Promix, vinylon, nylon, triacetate, polychlal, acrylic, polyurethane, polylactic acid, polyester, polyethylene, polypropylene, vinylidene, and polyvinyl chloride. On the other hand, examples of fibers having the official moisture regain higher than 5% may include wool, hemp, silk, rayon, polynosic, cupro, cotton, and acetate. In the present invention, "mainly contain" means to contain 50 mass% or more. Thus, assuming that a total mass of the surface layer 10 is 100%, 50 mass% or more thereof is non-absorbent fibers, and assuming that a total mass of the absorbent layer 20 is 100%, 50 mass% or more thereof is non-absorbent fibers. According to the present aspect, the surface layer 10 and the absorbent layer 20 mainly contain the non-absorbent fibers, so that the fibers themselves hardly retain the body fluid, and diffusibility of the body fluid can be improved as compared with a configuration mainly containing absorbent fibers. The surface layer 10 may preferably contain 75 mass% or more of non-absorbent fibers, may more preferably contain 90 mass% of non-absorbent fibers, or may be constituted of only non-absorbent fibers (without containing absorbent fibers). Similarly, the absorbent layer 20 may preferably contain 75 mass% or more of non-absorbent fibers, may more preferably contain 90 mass% of non-absorbent fibers, or may be constituted of only non-absorbent fibers (without containing absorbent fibers).

Water repellency of a skin surface of the surface layer 10 is higher than water repellency of a non-skin surface of the surface layer 10. According to the present aspect, the water repellency of the non-skin surface of the surface layer 10 is relatively low, so that the body fluid drawn into the surface layer 10 can be easily guided to the absorbent layer 20. Additionally, the water repellency of the skin surface of the surface layer 10 is relatively high, so that the body fluid once drawn into the surface layer 10 can be prevented from returning. The water repellency can be compared depending on magnitude of a contact angle. The contact angle can be measured, for example, by the following method. (1) A test piece is allowed to stand for 24 hours in a constant temperature and humidity chamber at a temperature of 20 ± 5°C and a humidity of 65 ± 5% RH. (2) A water contact angle of the test piece is measured in accordance with "6. Sessile drop method" in JIS R 3257: 1999 "Testing method of wettability of glass substrate". As a contact angle measuring device, an automatic contact angle meter CA-V type manufactured by Kyowa Interface Science Co., Ltd. can be exemplified. (3) The measurement is performed ten times at different positions of the test piece, and an average value thereof is adopted as the contact angle. Depending on the water repellency of the test piece, an amount of water droplets and a measurement time after the water droplets are placed on the test piece may be changed. A configuration in which the water repellency of the skin surface of the surface layer 10 and the water repellency of the non-skin surface of the surface layer 10 are made different from each other is not particularly limited. For example, water-repellent treatment such as application of a liquid repellent may be performed on the skin surface of the surface layer 10, hydrophilic treatment may be performed on the non-skin surface of the surface layer 10, or both of water-repellent treatment on the skin surface of the surface layer 10 and hydrophilic treatment on the non-skin surface of the surface layer 10 may be performed. On the skin surface of the surface layer 10, a region having higher water repellency than that of the non-skin surface of the surface layer 10 constitutes a water-repellent region R10 shown in FIG. 6(a). The water-repellent region R10 may be a region subjected to water-repellent processing. More preferably, the water repellency may be measured and compared by the following method. Five sheets of filter paper are stacked and placed on a horizontal stand, and a sample is placed thereon. Using a microsyringe, 5 µL of ion exchanged water is dropped. After standing for 5 seconds from completion of the dropping, it is checked whether the sample transmits or absorbs the ion exchanged water. This process is performed at 20 locations, and the number of locations where the ion exchanged water is transmitted or absorbed is counted. The water repellency is higher as the number thereof is smaller, and the water repellency is lower as the number thereof is larger.

The absorbent layer 20 includes a drawing-in region 25 in which density increases from the skin side T1 toward the non-skin side T2. The drawing-in region 25 is a region within a certain range extending in the thickness direction T, and is constituted of a low-density portion 21 having relatively low density, and a high-density portion 22 which is located on the non-skin side T2 relative to the low-density portion 21 and has higher density than that of the low-density portion 21. The drawing-in region 25 includes a diffusion portion 27 containing fibers orientated in a plane direction. The diffusion portion 27 contains fibers oriented in the plane direction, and can easily diffuse the body fluid in the plane direction. The body fluid guided into the absorbent layer 20 is promoted to move from the low-density portion 21 to the high-density portion 22 in the drawing-in region 25, is diffused in the plane direction by the fibers oriented in the plane direction of the diffusion portion while being guided to the non-skin side, and is retained over a wide range. It is possible to suppress liquid returning caused by locally retaining the body fluid. The diffusion portion 27 may partially contain fibers oriented in the thickness direction T and the like, or may be mainly constituted of fibers oriented in the plane direction. The diffusion portion 27 may be part of the drawing-in region 25, or may be the whole of the drawing-in region 25. Preferably, the diffusion portion 27 may mainly contain fibers oriented in the plane direction.

The body fluid discharged to the skin surface of the surface layer 10 is guided to the absorbent layer 20 from the surface layer 10. At this time, the water repellency of the non-skin surface (surface on the absorbent layer 20 side) of the surface layer 10 is relatively low, whereby the body fluid is likely to be quickly and smoothly guided from the surface layer 10 to the absorbent layer 20. Since the surface layer 10 and the absorbent layer 20 are mainly constituted of non-absorbent fibers, the surface layer 10 and the absorbent layer 20 can suppress retention of the body fluid and promote diffusion of the body fluid as compared with the shorts 1 mainly constituted of absorbent fibers, and it is possible to suppress liquid returning caused by local retention of the body fluid. The surface layer 10 is mainly constituted of non-absorbent fibers, and is less likely to retain liquid. Thus, by quickly guiding the body fluid from the surface layer 10 toward the absorbent layer 20, it is possible to improve a feeling on the skin and a feeling of attachment. The body fluid that has reached the inside of the absorbent layer 20 is guided to the non-skin side T2 by the drawing-in region 25. Due to this, the body fluid is less likely to be retained at a part close to the surface layer 10 in the absorbent layer 20, and the body fluid can be prevented from returning to the surface layer 10 side. Since the diffusion portion 27 is disposed in the drawing-in region 25, the body fluid can be diffused over a wide range in the plane direction in a process of guiding the body fluid to the non-skin side T2 or after guiding the body fluid to the non-skin side T2. By retaining the body fluid in the wide range in the plane direction of the absorbent layer 20, it is possible to suppress liquid returning caused by locally retaining the body fluid.

The official moisture regain of the non-absorbent fibers of the surface layer 10 may be less than 1%. The fibers having the official moisture regain of less than 1% have a small amount of moisture during normal use such as during wearing, suppress stickiness of the surface layer 10 that comes into contact with the skin, and can improve a feeling of attachment. The fibers having the official moisture regain of less than 1% hardly retain the body fluid, so that diffusion of the body fluid can be further promoted, and a drawing-in property can be improved. The surface layer 10 is mainly constituted of non-absorbent fibers, and the non-absorbent fibers may contain fibers having the official moisture regain of less than 1%. Thus, the surface layer 10 may contain fibers having the official moisture regain of 1% or more. Preferably, assuming that a total of the non-absorbent fibers of the surface layer 10 is 100 mass%, fibers having the official moisture regain of less than 1% may account for 50 mass%. Preferably, the non-absorbent fibers of the surface layer 10 may contain 75 mass% or more of fibers having the official moisture regain of less than 1%, more preferably may contain 90 mass% or more of fibers having the official moisture regain of less than 1%, or may be constituted of only non-absorbent fibers having the official moisture regain of less than 1% (without containing fibers having the official moisture regain of 1% or more). Examples of fibers having the official moisture regain of less than 1% include polyurethane, polylactic acid, polyester, polyethylene, polypropylene, vinylidene, and polyvinyl chloride. The fibers of the surface layer 10 may be 100 mass% of polyester (official moisture regain of 0.4%). Alternatively, the fibers of the surface layer 10 may be 100 mass% of a combination of nylon (official moisture regain of 4.5%) and polyurethane (official moisture regain of 1%). That is, the fibers of the surface layer 10 may be 100 mass% of non-absorbent fibers.

The official moisture regain of the non-absorbent fibers of the absorbent layer 20 may be less than 1%. The fibers having the official moisture regain of less than 1% hardly retain the body fluid, so that diffusion of the body fluid can be further promoted, and the drawing-in property can be improved. The absorbent layer 20 is mainly constituted of non-absorbent fibers, and the non-absorbent fibers may contain fibers having the official moisture regain of less than 1%. Thus, the absorbent layer 20 may contain fibers having the official moisture regain of 1% or more. Preferably, assuming that a total of the non-absorbent fibers of the absorbent layer 20 is 100 mass%, fibers having the official moisture regain of less than 1% may account for 50 mass%. Preferably, the non-absorbent fibers of the absorbent layer 20 may contain 75 mass% or more of fibers having the official moisture regain of less than 1%, more preferably may contain 90 mass% or more of fibers having the official moisture regain of less than 1%, or may be constituted of only non-absorbent fibers having the official moisture regain of less than 1% (without containing fibers having the official moisture regain of 1% or more). The fibers of the absorbent layer 20 may be 80 mass% of polyester (official moisture regain of 0.4%) and 20 mass% of nylon (official moisture regain of 4.5%), and may be 100 mass% in total of the both fibers. In the surface layer 10 in the present embodiment, fibers of the low-density portion 21 described later are 100 mass% of polyester (official moisture regain of 0.4%), and fibers of the high-density portion 22 described later are 80 mass% of polyester (official moisture regain of 0.4%) and 20 mass% of nylon (official moisture regain of 4.5%). That is, the fibers of the absorbent layer 20 may be 100 mass% of non-absorbent fibers.

In a plan view of the shorts, an area of the water-repellent region R10 on the skin surface of the surface layer 10 may be 50% or more and 90% or less of the entire skin surface of the surface layer 10. According to the present aspect, an area ratio of the water-repellent region R10 is equal to or larger than 50%, so that liquid returning can be suppressed in about half of the region. Since the area ratio of the water-repellent region R10 is equal to or smaller than 90%, the body fluid can be drawn into the non-skin side T2 at least in 10% of the region, and the drawing-in property of the body fluid can be secured.

As shown in FIG. 6(a), the surface layer 10 may include a plurality of projecting parts 11 projecting toward the skin side T1, and recessed parts 12 arranged between the projecting parts 11. The water-repellent regions R10 may be disposed at the projecting parts 11. More specifically, the water-repellent regions R10 may be disposed at top parts (part projecting toward the skin side) of the projecting part 11. Preferably, the water-repellent regions R10 may be disposed only at the projecting parts 11, and do not need to be disposed at the recessed parts 12. The projecting parts 11 project toward the skin side T1, and is likely to come into contact with the skin. The water-repellent regions R10 are disposed at the projecting parts 11 that come into contact with the skin, so that liquid hardly remains at a part that comes into contact with the skin, and a feeling of attachment can be improved. Additionally, liquid returning is hardly caused at the part that comes into contact with the skin, so that it is possible to prevent returned body fluid from coming into contact with the skin.

As shown in FIG. 6(b), the projecting parts 11 and the recessed parts 12 may be alternately arranged at intervals in a plan view. For example, the projecting parts 11 and the recessed parts 12 may be in rows, and the rows may be alternately arranged side by side. However, preferably, the recessed parts 12 may be arranged at intervals, and may be surrounded by the projecting parts 11. The projecting parts 11 surrounding the recessed parts 12 are continuous, and the recessed parts may be arranged at intervals from each other within the projecting parts 11. Since the recessed parts 12 are disposed in a region surrounded by the projecting parts 11, the body fluid can be guided from the surrounding projecting parts 11 to the recessed parts 12, and the body fluid can be moved to the non-skin side T2. The recessed parts 12 are surrounded by the projecting parts 11. The projecting parts 11 projecting toward the skin side T1 come into contact with the skin, and the recessed parts 12 hardly come into contact with the skin. Thus, the body fluid guided to the recessed part 12 as a non-water-repellent region hardly comes into contact with the skin, and a feeling of attachment can be improved.

As shown in FIG. 6(a), the absorbent layer 20 may be a knitted fabric in which fibers interwoven with a base material extending in the plane direction project in the thickness direction. The fibers projecting in the thickness direction T are continuous, and are arranged in a loop shape.

With the absorbent layer 20 as a knitted fabric in which the fibers project in the thickness direction T, the thickness of the absorbent layer 20 can be easily secured, and performance of retaining the body fluid can be improved. Additionally, since the absorbent layer 20 is a knitted fabric, it is easily stretched, and a property of following the body can be improved. In the knitted fabric, interwoven fibers are continuously arranged, and diffusibility of the body fluid can be improved.

The fibers constituting the knitted fabric may be modified cross-section fibers each having a diameter smaller than 10 µ. The modified cross-section fiber is a fiber whose cross section is not a substantially circular shape, which is divided or chipped, and differs in cross section depending on the fiber. More specifically, the modified cross-section fiber has a V-shaped groove extending in an axial direction on a fiber cross section, the groove becomes shorter toward a distal end of the fiber, and an inter-fiber distance becomes shorter. Due to this, there are variations in the inter-fiber distance as compared with fibers having the same cross sections, and diffusibility of the body fluid can be improved.

In the absorbent layer 20 of the present embodiment, loop-shaped fibers are arranged on each of the skin side T1 and the non-skin side T2 with respect to the sheet-like base material. A part where the loop-shaped fibers are arranged has lower density as compared with the base material, and constitutes the low-density portion 21. The base material has higher density as compared with the part where the loop-shaped fibers are arranged, and constitutes the high-density portion 22. In a region combining the high-density portion 22 constituted of the base material and the low-density portion 21 located on the skin side T1 relative to the base material, the density increases from the skin side T1 toward the non-skin side, and this region constitutes the drawing-in region 25.

The high-density portion 22 of the drawing-in region 25 may be disposed in a center region of the three equally divided regions obtained by dividing the absorbent layer 20 in the thickness direction. Since the high-density portion 22 is disposed in the center region in the thickness direction T of the absorbent layer 20, even if the body fluid is guided to the high-density portion 22, it is distant from the non-skin surface of the absorbent layer 20, and leakage from the non-skin surface of the absorbent layer 20 can be suppressed. Preferably, the high-density portion 22 may be disposed only in the center region. The low-density portions may be disposed on both of the skin side T1 and the non-skin side T2 of the high-density portion 22. With these configurations, leakage of the body fluid from the non-skin surface of the absorbent layer 20 can be further suppressed.

The high-density portion 22 may constitute the diffusion portion 27. That is, the high-density portion 22 mainly contains fibers oriented in the plane direction. Since the high-density portion 22 constitutes the diffusion portion 27, the body fluid moved from the low-density portion 21 to the high-density portion can be diffused in the plane direction. By guiding the body fluid to the non-skin side T2 and diffusing it in the plane direction at the high-density portion 22 located on the non-skin side T2, the drawing-in property in the thickness direction T of the absorbent layer 20 can be improved, and liquid residue and liquid returning on the skin surface of the shorts 1 can be suppressed.

The shorts 1 may include a crotch joining portion 40 that joins the surface layer 10 with the absorbent layer 20. The crotch joining portion 40 is a portion in which the surface layer 10 is joined with the absorbent layer 20, and examples thereof may include, for example, a sewn portion and a welded (thermally welded, ultrasonic welded) portion. The crotch joining portion 40 may join at least the non-skin surface of the surface layer 10 with the skin surface of the absorbent layer 20, or may join the entire region in the thickness direction T of the surface layer 10 with the entire region in the thickness direction T of the absorbent layer 20. The crotch joining portion 40 in the present embodiment is constituted of a portion in which the surface layer 10 and the absorbent layer 20 are sewn together over the entire thickness direction T. The crotch joining portion 40 joins the surface layer 10 with the absorbent layer 20, and does not join the waterproof layer 30 therewith. Density of the crotch joining portion 40 may be higher than that of a surrounding of the crotch joining portion 40. Since the density of the crotch joining portion 40 is higher than that of the surrounding, the body fluid can be easily guided to the crotch joining portion 40, movement of the body fluid from the surface layer 10 to the crotch joining portion 40 side can be promoted, and transferability of the body fluid to the absorbent layer 20 can be improved.

The density of the entire absorbent layer 20 may be lower than the density of the entire surface layer 10. According to the present aspect, since the density of the entire absorbent layer 20 is relatively low, the absorbent layer 20 can be easily dried when being washed for repeated use, and repeated use can be facilitated. As a modification, the density of the entire surface layer 10 may be lower than the density of the entire absorbent layer 20. According to this modification, liquid is likely to transfer from the surface layer 10 side where the density is relatively low to the absorbent layer 20 side where the density is relatively high, and the drawing-in property of the body fluid to the non-skin side can be improved. An average fiber diameter of the absorbent layer may be smaller than an average fiber diameter of the surface layer. A fiber diameter of the absorbent layer is relatively small, and the density of the absorbent layer is configured to be low.

Liquid diffusibility in the front-rear direction L of the absorbent layer 20 may be higher than liquid diffusibility in the width direction W of the absorbent layer 20. By diffusing the body fluid in a wide range in the front-rear direction L of the absorbent layer 20, the body fluid can be retained over a wide area, and liquid returning caused by local retention of liquid can be suppressed. The liquid diffusibility can be compared by dropping colored water and comparing areas (diffusion regions) where the colored water is diffused.

Subsequently, the following describes a configuration of suppressing leakage of the body fluid while enhancing fitness of the shorts 1 configured as described above to the body at the crotch region CR in detail. In the crotch region CR, there are disposed the crotch joining portion 40, and a crotch non-joining portion 45 in which the surface layer 10, the absorbent layer 20, and the waterproof layer 30 are not joined with each other. The crotch joining portion 40 joins only the surface layer 10 and the absorbent layer 20 among the surface layer 10, the absorbent layer 20, and the waterproof layer 30. The crotch joining portion 40 and the crotch non-joining portion 45 are disposed in the interior material 52. The crotch joining portion 40 may join at least the surface layer 10 and the absorbent layer 20. In a form in which another constituent element other than the surface layer 10 and the absorbent layer 20 is provided on the skin side T1 relative to the waterproof layer 30, the crotch joining portion 40 may join the other constituent element together. The crotch non-joining portion 45 is a region where the surface layer 10, the absorbent layer 20, and the waterproof layer 30 are not joined with each other, and may be disposed to be adjacent to the crotch joining portion 40. The crotch joining portions 40 are arranged on both sides in a first direction across the crotch center CRC. The first direction is one of the front-rear direction L and the width direction W. The crotch non-joining portion 45 is arranged overlapping with the pressing portion 60 between the crotch joining portions 40 in the first direction. The pressing portion 60 may overlap with at least part of the crotch non-joining portion 45.

By providing the crotch joining portions 40, misalignment of the surface layer 10 that comes into contact with the body in the crotch region CR can be suppressed, and fitness of the surface layer 10 can be improved. The crotch joining portions 40 are arranged on both sides across the crotch center CRC, and the surface layer 10 is not displaced from the absorbent layer 20 on both sides across the crotch center CRC, so that misalignment of the surface layer 10 at the crotch center CRC can be suppressed. The crotch joining portion 40 has higher rigidity as compared with the crotch non-joining portion 45. Thus, by disposing the crotch joining portion 40, twisting of the crotch region CR can be suppressed, and the crotch region CR can be continuously in contact with the body to suppress leakage of the body fluid. As shown in FIG. 7, during wearing, the crotch non-joining portion 45 arranged between the crotch joining portions 40 is likely to be lifted toward the wearer side with the crotch joining portions 40 as base points, and the absorbent layer 20 and the surface layer 10 are pushed up toward the body side by the pressing portion 60, so that fitness to the body can be improved. The crotch joining portion 40 joins only the surface layer 10 and the absorbent layer 20 among the surface layer 10, the absorbent layer 20, and the waterproof layer 30, and does not join the waterproof layer 30 therewith. Due to this, the body fluid absorbed by the surface layer 10 and the absorbent layer 20 can be prevented from reaching the waterproof layer 30 via the crotch joining portion 40, and leakage of the body fluid can be suppressed.

On the outer edge part of the crotch region CR, an outer edge joining portion that joins the surface layer 10, the absorbent layer 20, and the waterproof layer 30 may be disposed. The outer edge joining portion may include a side part joining portion 48 that joins the surface layer 10, the absorbent layer 20, and the waterproof layer 30 at the outer side portion of the interior material 52, and an end part joining portion 49 that joins the surface layer 10, the absorbent layer 20, and the waterproof layer 30 at the outer end of the interior material 52. The side part joining portion 48 and the end part joining portion 49 are located on the non-skin side relative to the skin surface of the shorts and on the skin side relative to the non-skin surface of the shorts, and are configured not to be seen when seen from the skin surface of the shorts and when seen from the non-skin surface of the shorts. The side part joining portion 48 and the end part joining portion 49 are not exposed at the skin surface and the non-skin surface of the shorts, so that it is possible to suppress a sense of incongruity that is caused when the outer edge joining portion comes into contact with the skin. The cross-sectional views in FIG. 4 and FIG. 5 show the side part joining portion 48 and the end part joining portion 49. The side part joining portion 48 extends in the front-rear direction L along the outer side portion of the interior material 52. The end part joining portion 49 extends in the width direction W at each of the front end and the rear end of the interior material 52. The side part joining portion 48 and the end part joining portion 49 are continuous to each other. Thus, the side part joining portion 48 and the end part joining portion 49 are arranged to surround the entire outer circumference of the interior material 52, and join the surface layer 10, the absorbent layer 20, and the waterproof layer 30 over the entire outer circumference of the interior material 52. In a region surrounded by the side part joining portion 48 and the end part joining portion 49, the crotch joining portion 40 and the crotch non-joining portion 45 are disposed. The waterproof layer 30 is not joined to the surface layer 10 and the absorbent layer 20 in a region other than the side part joining portion 48 and the end part joining portion 49. Configurations of the side part joining portion 48 and the end part joining portion 49 will be described later in detail. The crotch joining portions 40 in the present embodiment are arranged as a pair at an interval in the width direction W. The crotch non-joining portion 45 in the present embodiment is disposed, in the width direction W, in a region between the side part joining portion 48 and the crotch joining portion 40 and in a region between the pair of crotch joining portions 40, and is continuously disposed, in the front-rear direction L, between the end part joining portion 49 located at the front end of the interior material 52 and the end part joining portion 49 located at the rear end of the interior material 52.

Preferably, the first direction may be the width direction W. That is, the crotch joining portions 40 may be arranged on both sides in the width direction W of the crotch center CRC, and the crotch non-joining portion 45 may be arranged between the crotch joining portions 40 in the width direction W. According to the present aspect, with the crotch joining portions 40 located on both sides in the width direction W of the crotch center CRC as base points, a protruding shape rising toward the skin side T1 can be formed at the center in the width direction W of the crotch region CR. The protruding shape is likely to be formed by the surface layer and the absorbent layer joined by the crotch joining portions 40. The protruding shape can improve fitness to a vaginal opening, improve the drawing-in property of the body fluid, and suppress side leakage. In a modification, the first direction may be the front-rear direction L. That is, the crotch joining portions 40 may be arranged on both sides in the front-rear direction L of the crotch center CRC, and the crotch non-joining portion 45 may be arranged between the crotch joining portions 40 in the front-rear direction L.

The crotch joining portions 40 and the crotch non-joining portion 45 may extend in the front-rear direction L. Since the crotch joining portions 40 and the crotch non-joining portion 45 extend in the front-rear direction, fitness to the body can be improved over a range that is continuous in the front-rear direction L. The crotch joining portions 40 extending in the front-rear direction L serve as continuous base points to cause a region sandwiched by the crotch joining portions in the width direction W to rise toward the skin side, and a protruding shape is likely to be continuously formed in the front-rear direction. Since the protruding shape propagates to the rear side, fitness to a cleft of buttocks can be improved, and rear leakage can also be suppressed.

The pair of crotch joining portions 40 may extend in parallel with each other. That is, a length in the width direction W of the crotch non-joining portion 45 between the crotch joining portions 40 may be constant. As shown in FIG. 3, a length in the width direction of the crotch region CR varies in the front-rear direction L. A length in the width direction of the crotch center region CR3 is short, and the length is increased from the crotch center region CR3 toward an outer side in the front-rear direction L. Thus, the length in the width direction W of the crotch non-joining portion 45 between the crotch joining portion 40 and the side part joining portion 48 may vary. The length in the width direction W of the crotch center region CR3 is short, and the length is increased from the crotch center region CR3 toward the outer side in the front-rear direction L. To cause the pair of crotch joining portions 40 to function more easily as the base points for forming the protruding shape, the interval in the width direction W between the pair of crotch joining portions 40 may be equal to or larger than 5 mm. The crotch joining portions 40 may be disposed on both sides in the width direction across the crotch center CRC, or may be disposed at a plurality of points, not limited to one point on each side, on each of both sides in the width direction across the crotch center CRC.

The crotch joining portions 40 may be continuous from the front end edge of the crotch region CR to the rear end edge of the crotch region CR. The crotch joining portion 40 can join the surface layer 10 with the absorbent layer 20 over the entire region in the front-rear direction L of the crotch region CR, and can suppress twisting of the surface layer 10 and the absorbent layer 20. By suppressing twisting of the surface layer 10, the surface layer 10 can be brought into contact with the crotch of the wearer, and the drawing-in property of the body fluid can be secured. Additionally, by suppressing twisting of the absorbent layer 20, an area for absorbing the body fluid can be secured. The crotch joining portion 40 in the present embodiment reaches the end part joining portion 49 located at the front end of the interior material 52, and reaches the end part joining portion 49 located at the rear end of the interior material 52.

The pressing portion 60 may extend in the front-rear direction L. According to the present aspect, the pressing portion 60, the crotch joining portion 40, and the crotch non-joining portion 45 extend in the same front-rear direction L, so that the protruding shape can be formed over a range that is continuous in the front-rear direction L, and fitness to the body can be further improved. The pressing portion 60 may be arranged at the center in the width direction of the crotch region. The center in the width direction W of the crotch region CR can be pushed up toward the skin side T1 by the pressing portion 60. Thus, fitness to the vaginal opening can be further improved, and fitness to the cleft of the buttocks can also be improved. The pressing portion 60 may be arranged at an interval from the crotch joining portion 40 in the width direction. The region pushed up into the protruding shape (region where the pressing portion 60 is disposed) and the regions serving as the base points for pushing up into the protruding shape (regions where the crotch joining portions 40 are arranged) are separated from each other in the width direction W, so that respective functions thereof can be easily exhibited. The interval between the pressing portion 60 and the crotch joining portion 40 may be, for example, equal to or larger than 10 mm and equal to or smaller than 40 mm, and preferably equal to or larger than 10 mm and equal to or smaller than 25 mm. In a form in which the pressing portion 60 has a certain width direction, the pressing portion 60 may be arranged while straddling the center in the width direction W of the crotch region CR. In a form in which the pressing portions 60 are arranged in the width direction at intervals within predetermined intervals, a collection of the pressing portions arranged at the intervals may be arranged while straddling the center in the width direction W of the crotch region CR. The predetermined interval between the pressing portions 60 constituting the collection may be equal to or smaller than 10 mm. The pressing portion may be joined to the first exterior material and the second exterior material 512 to have a length equal to or larger than 85% and equal to or smaller than 95% of the length before being joined. The length in the width direction W of the pressing portion 60 may be equal to or larger than 5 mm and equal to or smaller than 20 mm in the crotch region CR. The length in the width direction W of the pressing portion 60 may be increased from the crotch region CR toward the rear side. A maximum length in the width direction of the pressing portion 60 in the rear-side region may be equal to or smaller than 55 mm. The pressing portion 60 in the present embodiment is flat rubber having a width of 10 mm, and is joined to the first exterior material 511 and the second exterior material 512 to have a length of 90% of the length before being joined. A material of the pressing portion 60 is not particularly limited, and rubber containing nylon and polyurethane can be exemplified, for example.

As shown in FIG. 5, a front end edge of the pressing portion 60 may be located on the rear side relative to the front end edge of the crotch region CR. That is, the pressing portion 60 does not need to be arranged at the front end edge of the crotch region CR. The front end edge of the pressing portion 60 may be arranged on the rear side relative to the front end edge of the crotch joining portion 40. Since the front end edge of the pressing portion 60 is located on the rear side relative to the front end edge of the crotch region CR, a region not overlapping with the pressing portion 60 is disposed at a front part of the absorbent layer 20. The front part of the absorbent layer 20 is less likely to be arranged facing the excretion opening, and has less necessity to be pressed against the body. By forming the front part of the absorbent layer 20 into a relatively flat shape, the body can be covered with the absorbent layer 20, and the body can be covered in a surface contact manner with the absorbent layer 20. In a region where the absorbent layer 20 overlaps with the pressing portion 60, the absorbent layer 20 is pressed against the body side due to expansion and contraction of the pressing portion 60, so that a gap between the body and the shorts can be reduced. Additionally, creases caused by expansion and contraction of the pressing portion 60 are less likely to be formed at the front part of the crotch region CR, so that appearance as the shorts can also be improved. A distance between the front end edge of the pressing portion 60 and the front end edge of the crotch region CR may be equal to or larger than 10 mm and equal to or smaller than 130 mm. Since the distance between the front end edge of the pressing portion 60 and the front end edge of the crotch region CR is equal to or larger than 10 mm, a region for forming a flat shape can be secured at the front part of the absorbent layer 20. Since the distance between the front end edge of the pressing portion 60 and the front end edge of the crotch region CR is equal to or smaller than 130 mm, fitness of the front part of the absorbent layer 20 can be secured, and front leakage can be suppressed.

A rear end edge of the pressing portion 60 may be arranged on the rear side relative to the rear end edge of the crotch region CR. The pressing portion 60 may be continuously disposed in the front-rear direction L while straddling the rear end edge of the crotch region CR. The pressing portion 60 extends toward the rear side relative to the rear end edge of the absorbent layer 20, so that the rear end edge of the absorbent layer 20 can be fitted to the body, and rear leakage can be suppressed. The rear end edge of the pressing portion 60 may extend to the waist portion 55. In a modification, the pressing portion 60 may be continuous from the front end edge of the crotch region CR to the rear end edge of the crotch region CR. By the pressing portion 60, the protruding shape can be formed over the entire region in the front-rear direction L of the crotch region CR, and fitness to the body can be further improved.

The surface layer 10, the absorbent layer 20, and the waterproof layer 30 are stretched or pressed following movement of the body during wearing, and repeatedly deformed during wearing. As a result of extensive studies conducted by the applicant, it has been found that, in a standing state or a sitting state of the wearer, the surface layer 10, the absorbent layer 20, and the waterproof layer 30 are likely to be stretched 1.1 times in the front-rear direction L assuming that a natural state is defined as 1.0 times. It has also been found that, in a state where the wearer is walking, exercising, or recrossing legs, the surface layer 10, the absorbent layer 20, and the waterproof layer 30 are likely to be stretched 1.5 times in the front-rear direction L assuming that the natural state is defined as 1.0 times.

Herein, 10% tensile strength of the surface layer 10 in the crotch center region CR3 is lower than the 10% tensile strength of the absorbent layer 20 in the crotch center region CR3, and is lower than the 10% tensile strength of the waterproof layer 30 in the crotch center region CR3. The 10% tensile strength in the crotch center region CR3 is tensile strength measured by the following method when the crotch center region CR3 is stretched in the front-rear direction L from 1.0 times to 1.1 times. The crotch center region CR3 is particularly required to have fitness to the excretion opening, and it is important to fit it into a gap of the body. The surface layer 10 is more likely to be stretched than the absorbent layer 20 in the crotch center region CR3, and is more likely to follow movement of the body. The surface layer 10 is likely to be deformed in a projecting shape due to expansion and contraction of the pressing portion 60, and is likely to be continuously fitted to the vaginal opening. The absorbent layer 20 is less likely to be stretched than the surface layer 10 in the crotch center region CR3, supports the surface layer 10 from the non-skin side T2 of the surface layer 10 to keep a shape of the surface layer 10, and can suppress leakage. The 10% tensile strength of the waterproof layer 30 in the crotch center region CR3 is lower than the 10% tensile strength of the absorbent layer 20 in the crotch center region CR3, so that the waterproof layer 30 is less likely to be stretched than the surface layer 10. Since the surface layer 10 is relatively likely to be stretched, the surface layer 10 follows the body, and since other members (the absorbent layer 20 and the waterproof layer 30) are less likely to be stretched, leakage can be suppressed while maintaining absorption capacity. The 10% tensile strength of the absorbent layer 20 in the crotch center region CR3 is higher than the 10% tensile strength of the surface layer 10 in the crotch center region CR3, and is higher than the 10% tensile strength of the waterproof layer 30 in the crotch center region CR3. In a low stretch state, the absorbent layer 20 is less likely to be stretched than the surface layer 10 and the waterproof layer 30, so that leakage can be suppressed while maintaining absorption capacity.

Herein, 50% tensile strength of the waterproof layer 30 in the crotch region CR is lower than the 50% tensile strength of the surface layer 10 in the crotch region CR, and may be lower than the 50% tensile strength of the absorbent layer 20 in the crotch region CR. The 50% tensile strength is tensile strength measured by the following method when the crotch region CR is stretched in the front-rear direction L from 1.0 times to 1.5 times. In a high stretch state (a state stretched 1.5 times), since the waterproof layer 30 is more likely to be stretched than the surface layer 10 and the absorbent layer 20, even when the shorts are largely stretched during exercise or the like, the waterproof layer 30 can follow movement of the body and continue to fit along the body, and leakage of the body fluid can be suppressed. In the high stretch state, since the surface layer 10 and the absorbent layer 20 are less likely to be stretched than the waterproof layer 30, the body can be covered while maintaining a fit shape formed in the low stretch state (a state stretched 1.1 times), so that absorption capacity can be maintained and leakage can be suppressed. The 50% tensile strength of the surface layer 10 in the crotch region CR may be lower than the 50% tensile strength of the absorbent layer 20 in the crotch region CR. When the shorts are largely stretched during exercise or the like, both of fitness and stability of absorption capacity are further required. Since the surface layer 10 is relatively likely to be stretched, the surface layer 10 can be flexibly fitted to movement of the body. Since the absorbent layer 20 is relatively less likely to be stretched, absorption capacity can be stably maintained.

The 10% tensile strength of the surface layer 10 in the crotch rear region CR2 is lower than the 10% tensile strength of the waterproof layer 30 in the crotch rear region CR2, and may be lower than the 10% tensile strength of the absorbent layer 20 in the crotch rear region CR2. The crotch rear region CR2 is a region that is likely to be deformed due to movement of the buttocks. Since the surface layer 10 is more likely to be stretched than the waterproof layer 30 and the absorbent layer 20, the surface layer 10 can follow movement of the body and maintain fitness to the buttocks. The absorbent layer 20 and the waterproof layer 30 are relatively less likely to be stretched, so that they can maintain the shapes thereof, can continuously cover the body, and can suppress leakage.

The 10% tensile strength of the exterior material 51 in the crotch region CR is lower than the 10% tensile strength of the interior material 52 in the crotch region CR. With this configuration, since the exterior material 51 is likely to be stretched, in the low stretch state at an initial stage of attachment, the absorbent layer 20 is pressed by the pressing portion 60 disposed in the exterior material 51, the protruding portion can be fitted to a recessed portion of the body such as the vaginal opening and the buttocks, and a structure that is less likely to cause leakage can be maintained.

Herein, the tensile strength can be measured by the following method. The tensile strength is measured by using a constant-rate extension type tensile tester. A sample to be measured is prepared. Specifically, five samples each having a width of 25 mm and a length of 160 mm are cut out in an MD direction and a CD direction. Measurement conditions are as follows: a tensile speed of 100 mm/min, a grip interval of 100 mm, and data: maximum strength, maximum tensile strength, and an optional tensile strength set as needed depending on the sample. The sample is correctly set in a grip portion of the tensile tester so that no load is applied to the sample. At this time, the grip interval is 100 mm, and a direction along the grip interval (direction of 100 mm) is aligned with a direction of 160 mm length of the sample. The sample is disposed to be stretched tight. It is assumed that strength when the sample is stretched by 10% (1.1 when the initial state is defined as 1.0) is 10% tensile strength, and strength when the sample is stretched by 50% (1.5 when the initial state is defined as 1.0) is 50% tensile strength. A unit is N/25 mm. In measuring the tensile strength of the crotch region, the grip interval is adjusted to grip a position adjacent to each outer end edge in the front-rear direction of the crotch region. In measuring the tensile strength of the crotch center region, the grip interval is adjusted to grip a position adjacent to each outer end edge in the front-rear direction of the crotch center region. In measuring the tensile strength of the crotch rear region, the grip interval is adjusted to grip a position adjacent to each outer end edge in the front-rear direction of the crotch rear region.

The crotch joining portion 40 may be a sewn portion in which the surface layer 10 and the absorbent layer 20 are sewn together. The sewn portion is a part that is sewn with threads and the like. The surface layer 10 and the absorbent layer 20 are brought into close contact with each other by threads or the like at the sewn portion, and the surrounding thereof is lifted in the thickness direction relative to the sewn portion. Thus, the crotch joining portion 40 constituted of the sewn portion is more likely to serve as the base point, and is likely to form a shape of the crotch non-joining portion 45 between the crotch joining portions 40 further rising toward the wearer side. In the sewn portion, holes through which threads pass are formed, so that the drawing-in property of the body fluid from the surface layer 10 to the absorbent layer 20 can be enhanced, and a feeling of attachment can be improved. The crotch joining portion 40 in the present embodiment is constituted of the sewn portion.

In a modification, the joining portion may be a bonding portion in which the non-skin surface of the surface layer 10 is bonded to the skin surface of the absorbent layer 20. The bonding portion includes welding by heat and the like, and bonding by a bonding agent. At the bonding portion, the non-skin surface of the surface layer 10 is bonded to the skin surface of the absorbent layer 20, and the skin surface of the surface layer 10 to the non-skin surface of the absorbent layer 20 are not constrained, so that degrees of freedom of the skin surface of the surface layer 10 and the non-skin surface of the absorbent layer 20 are increased. Due to this, it can easily follow movement of the body, and fitness to the body and an effect of continuously covering the body can be easily obtained. An area of the bonding portion constituted of the bonding agent is likely to be larger as compared with the sewn portion. Accordingly, an area of the joining portion can be secured, the surface layer 10 and the absorbent layer 20 can be joined over a wide range, and twisting and misalignment can be suppressed.

Subsequently, with reference to FIG. 4 and FIG. 5, the following describes configurations of the end part joining portion 49 and the side part joining portion 48 in detail. A surface waterproof layer that covers the skin side T1 of the surface layer 10 may be disposed in the crotch region CR. The surface waterproof layer may be part of a member constituting the waterproof layer 30 located on the non-skin side T2 relative to the absorbent layer 20, or may be separated from the member constituting the waterproof layer 30 located on the non-skin side T2 relative to the absorbent layer 20. In a region where the surface waterproof layer is arranged, the surface layer 10 and the absorbent layer 20 are sandwiched by the waterproof layer 30 in the thickness direction T. The surface waterproof layer can cover the skin side of the surface layer, and the waterproof layer can cover the non-skin side of the surface layer, so that it is possible to suppress leakage of the body fluid from the surface layer to the skin side, and leakage of the body fluid from the surface layer to the non-skin side.

The surface waterproof layer may include side part surface waterproof layers 31 arranged on both outer side portions of the interior material 52, and end part surface waterproof layers 32 arranged respectively at the front end and the rear end of the interior material 52. In the side part surface waterproof layer 31 in the present embodiment, the outer side portion of the waterproof layer 30 is folded toward the inner side in the width direction W with a first fold line FL1 as a base point located at the outside edge of the crotch region CR, and covers the skin side T1 of the surface layer 10 and the skin side of the side part joining portion 48. In a modification, the side part surface waterproof layer 31 may be constituted of a member different from the waterproof layer 30. The end part surface waterproof layer 32 in the present embodiment is constituted of a member different from the waterproof layer 30, and covers the skin side T1 of the surface layer 10 and the skin side T1 of the end part joining portion 49. In a modification, the end part surface waterproof layer 32 may be a part of the outer end of the waterproof layer 30 that is folded toward the inner side in the front-rear direction L with a fold line as a base point located at the outer end edge of the crotch region CR. Since the side part surface waterproof layer 31 wraps around outer edges of the surface layer 10 and the absorbent layer 20, and covers the skin surface of the surface layer 10 and the non-skin surface of the absorbent layer 20 with the waterproof layer 30, leakage from the outer edge part of the crotch region CR can be suppressed. The side part surface waterproof layers 31 and the end part surface waterproof layers 32 may be continuous to each other. Thus, the side part surface waterproof layer 31 and the end part surface waterproof layer 32 are arranged to surround the entire outer circumference of the surface layer 10 and the entire outer circumference of the absorbent layer 20, so that leakage of the body fluid from the outer edge of the surface layer 10 and the outer edge of the absorbent layer 20 can be suppressed.

The surface waterproof layer may include a first waterproof layer 35 constituting the skin surface of the shorts, and a second waterproof layer 36 that is continuous to the first waterproof layer 35 via a fold line and is located on the non-skin side T2 relative to the first waterproof layer 35. The second waterproof layer 36 is arranged between the first waterproof layer 35 and the surface layer 10. As shown in FIG. 4, the first waterproof layer 35 of the side part surface waterproof layer 31 is a region between the first fold line FL1 and a third fold line FL3 in the width direction W. The third fold line FL3 constitutes an inside edge of the first waterproof layer 35 of the side part surface waterproof layer 31 and an inside edge of the second waterproof layer 36 of the side part surface waterproof layer 31. An outside edge of the second waterproof layer 36 of the side part surface waterproof layer 31 is located on the inner side in the width direction W relative to the first fold line FL1. As shown in FIG. 5, the first waterproof layer 35 of the end part surface waterproof layer 32 is a region between the outer end edge of the surface waterproof layer and a fourth fold line FL4. The fourth fold line FL4 constitutes an inner end edge of the first waterproof layer 35 of the end part surface waterproof layer 32 and an inner end edge of the second waterproof layer 36 of the end part surface waterproof layer 32. The outer end edge of the second waterproof layer 36 of the end part surface waterproof layer 32 is located on the inner side in the width direction W relative to the outer end edge of the surface waterproof layer.

The outer edge joining portion may include a first outer edge joining portion 46 in which both of the first waterproof layer 35 and the second waterproof layer 36 are joined, and a second outer edge joining portion in which only the second waterproof layer 36 of the first waterproof layer 35 and the second waterproof layer 36 is joined. The first outer edge joining portion 46 and the second outer edge joining portion are sewn portions in which the surface layer 10, the absorbent layer 20, the first waterproof layer 35, and the second waterproof layer 36 are sewn together. As shown in FIG. 3 and FIG. 5, the first outer edge joining portion 46 joins the surface layer 10, the absorbent layer 20, the first waterproof layer 35, and the second waterproof layer 36. The first outer edge joining portion 46 does not join the waterproof layer 30 that is located on the non-skin side T2 relative to the absorbent layer 20. The first outer edge joining portion 46 in the present embodiment is disposed in a region between the outer end edge of the end part surface waterproof layer 32 and the fourth fold line FL4. The second outer edge joining portion corresponds to the side part joining portion 48 and the end part joining portion 49. As shown in FIG. 4, the side part joining portion 48 joins the surface layer 10, the absorbent layer 20, and the second waterproof layer 36, and does not join the first waterproof layer 35 and the waterproof layer 30 located on the non-skin side T2 relative to the absorbent layer 20. The end part joining portion 49 joins the surface layer 10, the absorbent layer 20, and the second waterproof layer 36, and does not join the first waterproof layer 35 and the waterproof layer 30 located on the non-skin side T2 relative to the absorbent layer 20.

Since the second outer edge joining portion is disposed, holes through which threads or the like pass are not formed, so that the body fluid once drawn into the absorbent layer 20 is less likely to return to the surface waterproof layer side, and liquid returning can be suppressed. On the other hand, holes are formed in the first outer edge joining portion 46, so that breathability and the drawing-in property from the surface waterproof layer side to the absorbent layer side can be improved. By disposing both of the first outer edge joining portion and the second outer edge joining portion, it is possible to improve the drawing-in property of the body fluid, the effect of suppressing returning of the body fluid, and the breathability. The outer edge joining portion is the sewn portion, so that the surrounding of the outer edge joining portion easily rises in the thickness direction with respect to the outer edge joining portion. Accordingly, a thickness of the surrounding of the outer edge joining portion is increased, and leakage from the outer edge part of the crotch region can be further suppressed. In a modification, the outer edge joining portion may include only the first outer edge joining portion 46, or may include only the second outer edge joining portion.

The first outer edge joining portion 46 may be arranged while straddling the center in the width direction W of the crotch region CR at the front end of the crotch region CR. At the first outer edge joining portion 46, the first waterproof layer 35 is sewn, and the first outer edge joining portion 46 can be seen from the skin side T1 of the shorts. The front end of the crotch region CR can be easily seen by the wearer when aligning positions during wearing, and the first outer edge joining portion 46 can be used as a mark for alignment. Particularly, when a pad such as a napkin is used in combination with the absorbent sanitary shorts, alignment of the pad can also be performed. The first outer edge joining portion in the present embodiment is disposed at the end part surface waterproof layer 32 located at the front end of the crotch region CR, and is not disposed at the end part surface waterproof layer 32 located at the rear end of the crotch region CR. The first outer edge joining portion in the present embodiment is not disposed at the side part surface waterproof layer 31. The side part surface waterproof layer 31 is a region that is likely to come into close contact with legs during wearing, so that it is possible to suppress a sense of incongruity that is caused when the sewn portion comes into contact with the legs.

The present invention has thus been explained in detail by using the above embodiment. It is, however, apparent to persons skilled in the art that the present invention is not limited to the embodiment described in the present specification. It is possible to carry out the present invention in revised or modified aspects, without departing from the gist and the scope of the present invention defined by the patent claims. Consequently, the description of the present specification aims to explain certain examples and is not intended to restrict the present invention.

The entire contents of Japanese Patent Application No. 2023-101024 filed on June 20, 2023, are incorporated herein by reference.

### [INDUSTRIAL APPLICABILITY]

It is possible to provide absorbent sanitary shorts that can suppress leakage of body fluid while enhancing fitness to a body at a crotch region.

### [REFERENCE SIGNS LIST]

1: absorbent sanitary shorts
10: surface layer
11: projecting part
12: recessed part
20: absorbent layer
21: low-density portion
22: high-density portion
25: drawing-in region
27: diffusion portion
30: waterproof layer
31: side part surface waterproof layer (surface waterproof layer)
32: end part surface waterproof layer (surface waterproof layer)
35: first waterproof layer
36: second waterproof layer
40: crotch joining portion
45: crotch non-joining portion
46: first outer edge joining portion
48: side part joining portion (second outer edge joining portion)
49: end part joining portion (second outer edge joining portion)
60: pressing portion
R10: water-repellent region
CR: crotch region
CR1: crotch front region
CR2: crotch rear region
CR3: crotch center region
CRC: crotch center
L: front-rear direction
T: thickness direction
T1: skin side
T2: non-skin side
W: width direction

## Claims

1. Absorbent sanitary shorts as reusable absorbent sanitary shorts comprising:
a surface layer; an absorbent layer arranged on a non-skin side relative to the surface layer; a waterproof layer arranged on the non-skin side relative to the absorbent layer; a crotch region arranged between a front end edge of the absorbent layer and a rear end edge of the absorbent layer; a crotch center located at a center in a width direction and a center in a front-rear direction of the crotch region; and a pressing portion arranged on the non-skin side relative to a non-skin surface of the absorbent layer to press the absorbent layer toward a skin side, wherein
crotch joining portions in which only the surface layer and the absorbent layer are joined among the surface layer, the absorbent layer, and the waterproof layer, and a crotch non-joining portion in which the surface layer, the absorbent layer, and the waterproof layer are not joined with each other are disposed in the crotch region,
the crotch joining portions are arranged on both sides in a first direction, which is one of the front-rear direction and the width direction, across the crotch center, and
the crotch non-joining portion is arranged overlapping with the pressing portion between the crotch joining portions in the first direction.

2. The absorbent sanitary shorts according to claim 1, wherein
the first direction is the width direction.

3. The absorbent sanitary shorts according to claim 2, wherein
the crotch joining portions and the crotch non-joining portion extend in the front-rear direction.

4. The absorbent sanitary shorts according to claim 3, wherein
the crotch joining portions are continuous from the front end edge of the crotch region to the rear end edge of the crotch region.

5. The absorbent sanitary shorts according to claim 3, wherein
the pressing portion extends in the front-rear direction.

6. The absorbent sanitary shorts according to claim 5, wherein
the pressing portion is arranged at the center in the width direction of the crotch region.

7. The absorbent sanitary shorts according to claim 5 or 6, wherein
a front end edge of the pressing portion is located on a rear side relative to a front end edge of the crotch region.

8. The absorbent sanitary shorts according to claim 5 or 6, wherein a rear end edge of the pressing portion is located on the rear side relative to a rear end edge of the crotch region.

9. The absorbent sanitary shorts according to any one of claims 1 to 6, wherein
50% tensile strength of the waterproof layer in the crotch region is lower than 50% tensile strength of the surface layer in the crotch region, and is lower than 50% tensile strength of the absorbent layer in the crotch region.

10. The absorbent sanitary shorts according to any one of claims 1 to 6, wherein
the crotch region includes a crotch center region located at a center of three equally divided regions obtained by dividing the crotch region in the front-rear direction, and
10% tensile strength of the surface layer in the crotch center region is lower than 10% tensile strength of the absorbent layer in the crotch center region, and is lower than 10% tensile strength of the waterproof layer in the crotch center region.

11. The absorbent sanitary shorts according to any one of claims 1 to 6, wherein
the crotch region includes a crotch rear region located on a rear side among three equally divided regions obtained by dividing the crotch region in the front-rear direction,
50% tensile strength of the waterproof layer in the crotch rear region is lower than 50% tensile strength of the surface layer in the crotch rear region, and
50% tensile strength of the surface layer in the crotch rear region is lower than 50% tensile strength of the absorbent layer in the crotch rear region.

12. The absorbent sanitary shorts according to any one of claims 1 to 6, wherein
the crotch joining portion is a sewn portion in which the surface layer and the absorbent layer are sewn together.

13. The absorbent sanitary shorts according to any one of claims 1 to 6, wherein
the crotch joining portion is a bonding portion in which a non-skin surface of the surface layer is bonded to a skin surface of the absorbent layer.

14. The absorbent sanitary shorts according to any one of claims 1 to 4, wherein
a surface waterproof layer that covers an outer edge part of the surface layer is disposed in the crotch region.

15. The absorbent sanitary shorts according to claim 14, wherein
the surface waterproof layer includes a first waterproof layer constituting a skin surface of the absorbent sanitary shorts, and a second waterproof layer that is continuous to the first waterproof layer via a fold line and is located on a non-skin side relative to the first waterproof layer,
an outer edge joining portion in which the surface layer, the absorbent layer, and the waterproof layer are joined is disposed at an outer edge part of the crotch region,
the outer edge joining portion includes a first outer edge joining portion in which both of the first waterproof layer and the second waterproof layer are joined, and a second outer edge joining portion in which only the second waterproof layer of the first waterproof layer and the second waterproof layer is joined, and
the first outer edge joining portion and the second outer edge joining portion are sewn portions in which the surface layer, the absorbent layer, and the waterproof layer are sewn together.

16. The absorbent sanitary shorts according to claim 15, wherein
the first outer edge joining portion is arranged while straddling the center in the width direction of the crotch region at a front end of the crotch region.

17. The absorbent sanitary shorts according to any one of claims 1 to 6, comprising:
an interior material including the surface layer, the absorbent layer, and the waterproof layer, and an exterior material arranged on a non-skin side relative to the interior material, wherein
the pressing portion is disposed in the exterior material, and
10% tensile strength of the exterior material in the crotch region is lower than 10% tensile strength of the interior material in the crotch region.

18. The absorbent sanitary shorts according to claim 17, wherein
the pressing portion is constituted of a non-elastic member arranged on a non-skin side relative to the absorbent layer.

19. The absorbent sanitary shorts according to any one of claims 1 to 6, wherein
the pressing portion is constituted of an elastic member arranged on a non-skin side relative to the absorbent layer.
